(19) **Europäisches Patentamt**
**European Patent Office**
**Office européen des brevets**

(11) **EP 2 601 608 B1**

(12) **EUROPEAN PATENT SPECIFICATION**

(45) Date of publication and mention
of the grant of the patent:
**09.10.2019 Bulletin 2019/41**

(21) Application number: **11755135.8**

(22) Date of filing: **05.08.2011**

(51) Int Cl.:
**G16H 50/70** *(2018.01)*   **G16H 15/00** *(2018.01)*

(86) International application number:
**PCT/IB2011/053509**

(87) International publication number:
**WO 2012/017418 (09.02.2012 Gazette 2012/06)**

(54) **REPORT AUTHORING**

BERICHTSAUTORISIERUNG

PUBLICATION DE RAPPORT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.08.2010 US 370832 P**

(43) Date of publication of application:
**12.06.2013 Bulletin 2013/24**

(73) Proprietor: **Koninklijke Philips N.V.
5656 AE Eindhoven (NL)**

(72) Inventors:
• **LEE, Michael, Chun-chieh**
  **NL-5656 AE Eindhoven (NL)**
• **COHEN-SOLAL, Eric**
  **NL-5656 AE Eindhoven (NL)**

(74) Representative: **Damen, Daniel Martijn et al
Philips Intellectual Property & Standards
High Tech Campus 5
5656 AE Eindhoven (NL)**

(56) References cited:
**EP-A1- 2 120 170      US-A- 6 047 259
US-A1- 2005 065 813**

**Description**

FIELD OF THE INVENTION

The invention relates to aiding report authoring.

BACKGROUND OF THE INVENTION

A Radiology Information System (RIS) integrated with Picture Archiving and Communication System (PACS) capabilities helps hospitals and imaging centers to optimize their workflow and manage images and information circulation throughout the facility to deliver efficient patient care and services. The different steps involved in this process start with a physician ordering a radiology procedure, a patient going to the radiology department, the imaging procedure being performed, a radiologist reading the images and producing a report to be sent to the referring physician.

The report that is produced by the radiologist typically summarizes relevant aspects of the patient history and the clinical question being posed, and then indicates what regions of the radiology image were examined and what findings there were, if any. The findings and any conclusions or impression that can be drawn from the findings are typically given in separate sections of the report. Broadly speaking, the ordering physician expects the findings and impression to address the clinical question that he or she has originally posed. If the findings and impression inadequately address the clinical question, it may be necessary for the ordering physician to contact the radiologist and ask for clarification.

Geis, J. (2007), "Medical Imaging Informatics: How It Improves Radiology Practice Today," Journal of Digital Imaging 20(2): 99 discloses that radiologists are under pressure to add more value to medical imaging-to provide more educated, accurate, useful, and efficient interpretations in the face of increasingly large and complex imaging studies and to communicate this information quickly and in the most useful manner. The radiology department and radiologist both need to be better, faster, and cheaper. Moreover, the radiologist synthesizes what he sees on the images, clinical data, and his medical knowledge to produce the interpretation. This should be a cohesive, accurate, helpful discussion that adds value to the images.

Therefore, there is a need for tools that can help them deliver the desired quality in reporting and still address the local constraints of each radiology institution. There is a need for consistency between radiologists in the way they report on similar cases (inter-radiologist consistency) as well as a need for consistency over time for each individual radiologist (intra-radiologist consistency). Moreover, the nature of their work imposes time constraints and reading quotas increasing the number of potentially incomplete and, consequently, potentially inaccurate reports. This inaccuracy will possibly lead to unnecessary interactions with referring physicians requesting further clarification, causing interruption and decrease of efficiency that can be avoided.

By way of background, US 2005/0065813A discloses an online medical evaluation system with interactive patient and physician interfaces. A patient enters symptoms and risk factors which are diagnosed by matching using a diagnostic tool based on medical knowledge, and the system can ask the patient questions to aid diagnosis.

SUMMARY OF THE INVENTION

The invention is defined in the claims.

It would be advantageous to have an improved aid for report authoring. To better address this concern, a first aspect of the invention provides a system comprising

- a set of associations, wherein an association links at least one first concept term in a knowledge domain with at least one second concept term in the knowledge domain, wherein the association indicates a usual co-occurrence of the at least one first concept term and the at least one second concept term in reports in the knowledge domain;
- a concept term extractor for extracting at least one first concept term relating to a particular subject from an at least partially completed report relating to the particular subject;
- a missing concept term finder for finding at least one second concept term linked to the at least one first concept term based on the set of associations, wherein the at least one second concept term is missing from the at least partially completed report;
- an indicator for indicating the found at least one second concept term to a user.

The system thus provides an indication of missing second concept terms which are associated with first concept terms relating to the subject. By providing this indication of missing second concept terms, the user is reminded to include a comment involving the second concept term in the report. This way, it is easier to author a complete report, because any potentially missing concept terms are indicated to the user. Consequently the number of incomplete reports may be reduced. Moreover, when the report is read by a consumer of the report, such as a referring physician, fewer interactions with the author of the report are necessary because the report contains the information about the relevant concept terms. Alternatively, when the concept was already discussed in the report using different terminology, the user may be reminded to rephrase the report to use the second concept term. This improves the consistency of the terminology used in the reports. The user of the system may comprise an author of the at least partially completed report.

The knowledge domain may comprise a medical knowledge domain, the subject may comprise a patient, and the report may comprise a medical diagnosis or treatment report. In an effort to help ensure that medical reports adequately address the clinical question posed by e.g. a referring physician, the system may provide means of verifying that a radiologist or physician completes the report using the terminology expected based on the clinical context.

The concept extractor may be arranged for extracting the at least one first concept term from the at least partially completed report. The first concept term(s) may be present in the at least partially completed report, for example, in an introductory section in which the context of the report is set forth. Such sections may include a clinical history description and/or a description of the questions asked by a referring person.

The concept extractor may be arranged for extracting the at least one first concept term from a first section of the at least partially completed report. Moreover, the set of associations may comprise associations linking first concept terms from the first section to second concept terms belonging to a different second section of the reports in the knowledge domain. This way, the concepts which need discussion in the second section of the report, based on the first section of the report, may be identified.

The concept extractor may be arranged for extracting the at least one first concept term from medical history information represented by the data record. Medical history information may provide clues to what concept terms should be discussed in the remainder of the report.

The at least one second concept term may be associated with a particular section of the report, and the indicator may be arranged for indicating with which section the at least one second concept term is associated. This makes the suggestion of missing concept terms more specific, because the missing concept terms are presented in the context of the section in which they should appear.

The system may comprise a report input for receiving a completed report, and wherein the at least partially completed report is the completed report. The completed report may thus be checked for missing concept terms. This way, the completed report may be verified. If any missing concept terms are identified, the completed report and the missing concept terms may be presented to the author (or to another author), who may then consider providing additional text involving the missing concept.

The system may comprise a report creation tool for enabling a user to create at least part of the report. The indicator may be arranged for indicating the found at least one second concept term during a process of creating the at least part of the report. For example, the indicator may be part of the report creation tool. After entering a word, the indicator may update the indication of missing concept terms. This helps the user to check the completeness of the report during creating the report, which helps creating reports having the necessary information and may avoid any iterations of correcting or extending the report.

The at least one second medical concept term may comprise an anatomical region term. The system may comprise an image viewer configured to display a view of an anatomical region of the subject based on the anatomical region term, wherein the anatomical region corresponds to the anatomical region term. This way, the number of manual interactions is reduced, because the relevant anatomical region is visualized automatically.

The system may comprise a second concept term classifier for classifying the at least one second concept term by type of concept, and wherein the indicator is arranged for indicating the type of concept for a second concept term. This provides a more organized view of the concepts.

The associations may have different strengths. The system may comprise a second concept term sorter for sorting the second concept terms found by the missing concept term finder, based on the strengths of the associations by which the second concept terms were found. This way, the concept terms which are most likely to be relevant for discussion in the report may be indicated as such to the user.

The system may comprise an association generator for creating an association based on co-occurrence frequencies of first and second concept terms in a collection of reports. This way, the set of associations can be generated automatically. Moreover, the set of associations can be updated taking into account new reports as they are added to the collection.

The system may be incorporated in a workstation, for example a medical workstation.

In another aspect, the invention provides a method of aiding report authoring, comprising

- extracting at least one first concept term relating to a particular subject from an at least partially completed report relating to the particular subject;
- finding at least one second concept term linked to the at least one first concept term based on a set of associations, wherein the at least one second concept term is missing from the at least partially completed report, wherein an association of the set of associations links at least one first concept term in a knowledge domain with at least one second concept term in the knowledge domain, wherein the association indicates a usual co-occurrence of the at least one first concept term and the at least one second concept term in reports in the knowledge domain; and

- indicating the found at least one second concept term to a user.

In another aspect, the invention provides a computer program product comprising instructions for causing a processor system to perform the method set forth.

It will be appreciated by those skilled in the art that two or more of the above-mentioned embodiments, implementations, and/or aspects of the invention may be combined in any way deemed useful.

Modifications and variations of the workstation, the method, and/or the computer program product, which correspond to the described modifications and variations of the system, can be carried out by a person skilled in the art on the basis of the present description.

BRIEF DESCRIPTION OF THE DRAWINGS

These and other aspects of the invention are apparent from and will be elucidated with reference to the embodiments described hereinafter. In the drawings,

Fig. 1 is a diagram of a system for aiding report authoring;
Fig. 2 is a flowchart of a method of aiding report authoring; and
Fig. 3 is a diagram of a system for aiding report authoring.

DETAILED DESCRIPTION OF EMBODIMENTS

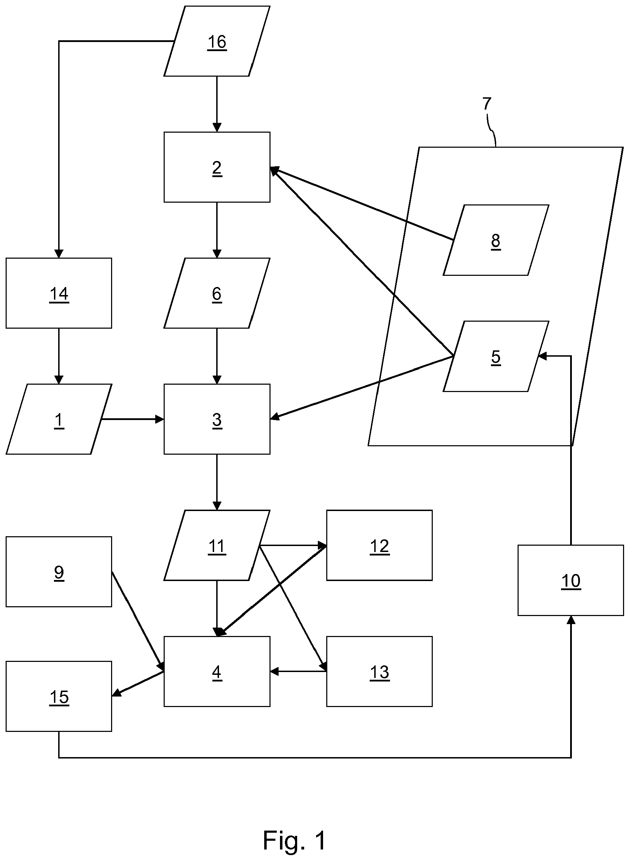

Fig. 1 illustrates a system for aiding report authoring. The system may in particular be used as an aid in authoring reports in a particular knowledge domain. Examples of such a knowledge domain include radiology, cardiology, oncology. It is possible to tailor the system for a narrower knowledge domain such as lung x-ray diagnosis reports, or for a broader knowledge domain, such as medical reports in general. Other, non-medical knowledge domains may also be applied. The Figure shows an exemplary embodiment with a number of features. These features are only described by means of example; most of the features are optional or can be implemented in different ways.

The system may comprise a set of associations 1. Each association in the set may link at least one first concept term in a knowledge domain with at least one second concept term in the knowledge domain. Associations may indicate that the at least one first concept term frequently co-occurs with the at least one second concept term in reports in the knowledge domain. In other words, reports containing the first concept term frequently also contain the second concept term. The set of associations 1 may be prepared in advance. It is also possible to update the set of associations 1 by analyzing a collection of reports, as will be described hereinafter. The concept terms which are used in the knowledge domain may be organized in a set of concept terms 16. The associations may also indicate that when a data record 7 contains a first concept term, the report frequently contains a second concept term.

The system may further comprise a concept term extractor 2 for extracting at least one first concept term 6 from a data record 7. The data record 7 may be part of a database with data records, wherein each data record may contain information relating to a particular subject, such as a patient. The data record 7 may be understood to also comprise any subject-related data files which are currently being edited, such as a partially completed diagnostic report which is loaded in a report editor. Accordingly, the data record 7 may comprise an at least partially completed report 5 relating to the particular subject. The concept term extractor may be arranged for extracting concept terms matching one of the concept terms in the set of concept terms 16. In particular, the concept term extractor 2 may be arranged for extracting any concept terms which are used as first concept terms in the set of associations 1.

The system may further comprise a missing concept term finder 3, for finding any concept terms which are missing in the at least partially completed report, but which would be expected to appear in the report based on the associations and the concept terms extracted from the data record 7. To this end, the missing concept term finder 3 may identify one or more concept terms which are linked to the at least one first concept term 6 extracted by the concept term extractor 2 by at least one of the associations 1. Moreover, the missing concept term finder 3 may check whether any of these one or more concept terms are missing from the at least partially completed report 5. The missing concept terms are outputted by the missing concept term finder as the at least one second concept term 11.

The system may further comprise an indicator 4 for indicating the found at least one second concept term 11 to a user. This indication can be performed in numerous ways. For example, the at least one second concept term 11 may be shown on a display in a list form. This may enable the user to update the at least partially completed report 5 with information including the at least one second concept term 11.

As discussed above, in medical applications, the knowledge domain may comprise a medical knowledge domain, the subject may comprise a patient, and the report may comprise a medical diagnosis or treatment report.

The concept term extractor 2 may be arranged for extracting the at least one first concept term 6 from the at least partially completed report 5. Consequently, it is possible that the concept term extractor 2 only evaluates the at least partially completed report, and not the remainder of the data record 7. This may be the case where the reports are self-contained, e.g., they contain the clinical history, any questions posed by the referring physician, the findings, and the diagnosis. These items may be organized in different sections of the report. Another division into sections may also be provided.

Such a systematic sectioning of reports may be used by the system. The concept term extractor 2 may be arranged for extracting the at least one first concept term 6 from a first section of the at least partially completed report 5. The set of associations 1 may comprise associations linking first concept terms belonging to the first section to second concept terms belonging to a different second section of the reports in the knowledge domain. Consequently, the missing concept term finder 3 may then find missing concepts in the second section of the report based on the concept terms extracted from the first section of the report. For example, the first section may comprise clinical history information and/or a diagnostic question posed, and the second section may comprise clinical findings and/or diagnosis.

The at least one second concept term 11 may be associated with a particular section of the report, in the sense that the at least one second concept term frequently appears in that particular section of reports in the knowledge domain. In such a case, the indicator 4 may be arranged for indicating with which section the at least one second concept term is associated. For example, the at least one second concept term may be displayed while the user is editing the relevant section of the report. When the user is editing another section, the second concept terms associated with that other section may be displayed. For example, while editing the "Findings" section, the second concept terms associated with the "Findings" section may be displayed; while editing the "Impressions" section, the second concept terms associated with the "Impressions" section may be displayed.

The concept term extractor 2 may be arranged for extracting the at least one first concept term 6 from medical history information 8 represented by the data record. This medical history information 8 may include past reports, medicine use, lab reports, and more.

The system may further comprise a user interface element 9 for enabling a user to indicate that the report 5 is complete, and wherein the indicator 4 is arranged for indicating the found at least one second concept term 11 in response to a user indication that the report 5 is complete. The concept term extractor and the missing concept term finder may also be triggered by the user indication, however they may also work in the background while the report is being written. The indication of missing concept terms to the user may be given only when the report 5 is considered complete, to avoid any biased reporting by the user.

The system may further comprise a report creation tool 10 for enabling a user to create the report 5 or at least part thereof. For example, an editor may be provided enabling a user to type in the (sections of the) report. The report creation tool 10 may also be based on speech recognition. The tool 10 may enable a user to dictate the report and extract the at least one first concept term 6 from the dictated text. Moreover, the missing concept term finder 3 may be arranged for checking whether the relevant concept terms are missing from the dictated text. The indicator 4 may be arranged for indicating the found at least one second concept term 11 during a process of creating the at least part of the report 5. Immediate feedback may guide a user to the relevant concepts.

The at least one second concept term 11 may comprise an anatomical region term, such as lungs or heart. The system may further comprise an image viewer 15 configured to indicate a view of the anatomical region corresponding to the anatomical region term. In particular, a medical image of the patient may be shown with an indication of the anatomical region. For example, the anatomical region may be indicated by highlighting or by means of an arrow.

The system may comprise a second concept term classifier 12 for classifying the at least one second concept term 11 by type of concept. Examples of types of concepts may include anatomical regions, diseases, findings, diagnosis. The indicator 4 may be arranged for indicating the type of concept for a second concept term 11. For example, the type of concept may be displayed next to the concept term. Alternatively, the second concept terms may be ordered in groups, each group corresponding to a concept type.

The associations of the set of associations 1 may have different strengths. The system may further comprise a second concept term sorter 13 for sorting the at least one second concept term 11 found by the missing concept term finder 3. The sorting allows the user's attention to be drawn to the most pertinent missing concept terms. The sorting may be based on the strengths of the associations by which the second concept terms 11 were found.

The system may further comprise an association generator 14 for creating an association based on co-occurrence frequencies of first and second concept terms in a collection of reports. These co-occurrence frequencies may be used to estimate a probability that a report containing a first concept term also contains a second concept term. When this probability is high enough, the association linking these concept terms may be stored in the set of associations 1. This will be further elucidated hereinafter. Other ways to obtain the set of associations 1 are also possible. For example, the associations could be manually created. The associations could also be derived from co-occurrence frequencies of first and second concept terms in a collection of textbooks or scholarly articles on the domain, for example by data-mining for co-occurrence frequencies in medical journals. This could be performed in addition to or instead of analyzing the collection of reports.

The system set forth may be implemented as a workstation loaded with a suitable application program.

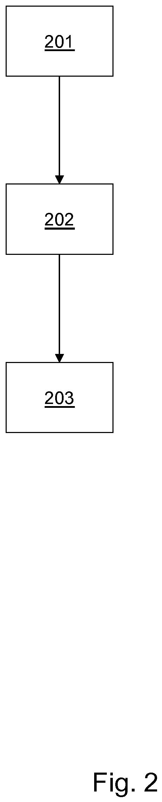

Fig. 2 illustrates a method of aiding report authoring. The method may comprise step 201 of extracting at least one first concept term relating to a particular subject from a data record associated with the particular subject, the data record comprising an at least partially completed report relating to the particular subject. The method may further comprise step 202 of finding at least one second concept term linked to the at least one first concept term based on a set of associations, wherein the at least one second concept term is missing from the at least partially completed report, wherein an association of the set of associations links at least one first concept term in a knowledge domain with at least one second concept term in the knowledge domain, wherein the association indicates a frequent co-occurrence of the at least one first concept term and the at least one second concept term in reports in the knowledge domain. The method further comprises step 203 of indicating the found at least one second concept term to a user. The method may be modified or extended, e.g. based on the functionality described herein in respect of the system. The method may be implemented as a computer program product comprising instructions for causing a processor system to perform the method.

In addition to radiology reports, the system may be used in other medical fields for which reports are routinely generated. Examples include but are not limited to cardiology, oncology, pathology, or surgery. As a concrete example for cardiology, a report for which "pericarditis" is the diagnosis may be reasonably expected to mention "friction rub" as one of the findings. A report lacking this term may be flagged, as it is either lacking a term that the reader might expect (i.e. the finding should be mentioned as being present or absent) or the terminology actually used in the report is somehow non-standard, and a more consistent use of terminology is appropriate.

One of the applications of the techniques described herein is to provide a functionality to discover and suggest relevant or missing medical concept terms to be possibly added to the report by looking at prior reports, being a source of a massive amount of medical practices information. The approach to create a set of associations 1 may comprise extracting key associations or correlations between the different pieces of information contained in prior reports. The key correlations that are used in this approach may comprise correlations between the patient clinical history (body location(s), symptoms, signs, reason(s) for the exam, prior knowledge) and radiologists' findings, reported anatomical regions, impressions and diagnosis. Other significant correlations between different findings within the report or between findings and diagnosis can be identified as well and used.

An example of such a correlation is the following. Consider a CT scan for a patient complaining of "tinnitus". Analysis of reports may show that it is customary for a radiologist to describe the patient's "auditory canals". In some cases, this may be to report no problems in that area, while in others a specific pathology is reported. However, a physician who has ordered this CT scan may reasonably expect to receive the CT report and find some indication of the status of the patient's "auditory canals".

The indication of missing concept terms may be used in a scenario where the radiologist is about to submit the patient case report and asks the system to perform a checking analysis (post-reporting checking). It is also envisioned to suggest possibly relevant medical terms earlier, during the report generation process itself as the radiologist is typing or dictating a report (with speech-to-text technology), or even earlier during the reading, interpretation process where recommendations can be suggested. As an example, giving recommendations to a resident in training can offer access to knowledge the resident does not already know or possess.

Following are brief descriptions of features in the context of an example of an envisioned scenario:

Post reporting checking: Suggesting medical terms or concepts to improve the clinical quality of the report when the report is completed. In this scenario the objective is to provide relevant, missing terms to address consistency, completeness and accuracy of each produced report. Given the clinical history information (body location(s), symptoms, signs, reason(s) for the exam, prior knowledge) for the current patient study, the system may suggest medical concept terms that are typically associated with this particular patient history but are not found in the final report. The radiologist can then read the suggestions and add terms to the report if necessary. The missing terms can be medical concepts related to findings, anatomical regions, impressions and diagnosis. In particular, consistency is addressed not necessarily by establishing a set of rules on reporting practices (from a given institution for example) that physicians have to comply to. It is done by providing a tool that seamlessly suggests medical terms without having to remember which rules apply to this particular patient case.

Reporting assistance: Suggesting medical terms during the creation of the patient report. As the physician is generating the report, medical terms may be displayed which are known to be associated with the available clinical history terms. At the beginning of the reporting, a list of terms is suggested. The radiologist can decide to look at these terms or not. As he is typing or dictating the report, more input terms become available to the system, allowing for more context-focused suggestions of medical terms (correlations between findings or between findings and diagnosis are used). It is also possible to provide an auto-completion function as the simple typing of the beginning of a word narrows down dramatically the possible terms to be proposed. In such a case, the system might have more influence in the selection of terms but offers the possibility to further clarify the description and, perhaps, to hint the radiologist to trigger a course of investigation not considered yet.

Region recommender through smart sorting during reading: The radiologist often knows which image regions to investigate to look for patterns or specific, abnormal visual characteristics, given the clinical history. One of the purposes may be to promote the most probable region terms, based on what has been reported in previous patient studies for a similar clinical history, and consequently avoid regions being overlooked. It also promotes unseen discovery of new links between for example a symptom and an anatomical region, that was not necessarily taught in medical textbooks or training.

The set of associations 1 may be created based on an analysis of prior reports. Such analysis may comprise the step of extraction of relevant pieces of information from radiology reports. Radiology reports are artifacts and end result of the radiology reading exercise. A radiology report contains the clinical history of the patient (body location(s), symptoms, signs, reason(s) for the exam, prior knowledge) and the radiologist's findings, relevant anatomy locations and conclusion with current diagnosis for the patient. The report can vary from well structured (form) to less structured (most of the cases today) with sections containing free text.

In the case of reports with free text, existing natural language processing (NLP) techniques can be used to automatically extract the relevant pieces of information in the form of medical concepts (or terms).

As an illustrative example of clinical history: "Patient with history of neurofibromatosis and hypothalamic astrocytoma. Patient with severe headaches on the right side where the shunt is located." In this case, the system may identify four key clinical history terms: astrocytoma, headache, shunt and neurofibromatosis. From the same report the system may extract medical concepts (terms) from both the findings and conclusion sections: basal ganglia, dentate nucleus, frontal lobe, hypothalamus, nerve, optic chiasm, septum pellucidum, soft tissue, third ventricle.

The analysis of prior reports may further comprise identification of the associations, correlations of medical terms. In this step, relevant statistics may be collected. These statistics may comprise the frequency of finding a given medical concept within all the reports and/or the co-occurrence frequency between two different types of medical concepts (a co-occurrence being defined as two different terms both appearing at least once in a single report). For example, the frequency with which a concept term appears in a clinical history may be determined as a term frequency, and the frequency with which a first concept term in a clinical history co-occurs with a second concept term in a finding/diagnosis may be determined as a co-occurrence frequency. Accordingly, the counting of the co-occurrences may be restricted based on the location of the terms in the report. For example, a co-occurrence between term A and term B may be counted only if term A appears in the "History" section of the report and term B appears only in the "Findings" section of the report. However, this is not a limitation. The counting of the co-occurrences may be weighted by whether a positive finding was detected. Moreover, the counting of the co-occurrences may be weighted by the seniority of the reading radiologist.

The analysis of prior reports may further comprise selection of significant associations, correlations of medical terms. Some of the identified correlations may be insignificant or just random. In an example embodiment, a Fisher's exact test is applied and the obtained p-value is used to sort the list of associations based on their significance, ranging from the most significant to the most probably random ones at the bottom of the list. Other statistical tests, such as Chi-square tests, are equally applicable.

As an example, consider a set of 800 reports, in which term A appears at least once in every 70 reports, and term B appears at least once in every 10 reports. If these terms appeared randomly in the reports (no real meaningful association), then on average one would expect about 2 out of the 800 reports to contain both term A and B. If, instead, one observes 8, or 9, or 10 reports with both terms, then there may be a good chance that this is a real meaningful association. A variety of well known statistical tests are available to discover this situation in a rigorous manner. The choice of a specific test is not meant to limit the invention.

The analysis of prior reports may further comprise estimation of conditional probabilities linking medical terms. In an example embodiment, the significant associations are found using conditional probabilities. The probabilities of interest here may include:

P(finding term | clinical history term), and/or
P(diagnostic term | clinical history term).

More generally, the conditional probability may be formulated as:
P(second concept term | first concept term).

A conditional probability may be estimated by taking the ratio of the co-occurrence frequency and the first concept term frequency using the Bayes' equation as follows:

$$P(\text{second concept term} \mid \text{first concept term}) =$$

$$P(\text{first concept term AND second concept term}) / P(\text{first concept term}) .$$

The co-occurrence frequency may be used to estimate P(first concept term AND second concept term). The occurrence frequency of the first concept term may be used to estimate P(first concept term). The same principle may be applied to compute P(finding term | clinical history term) and/or P(diagnostic term | clinical history term).

It is also possible to estimate multi-term conditional probabilities, such as P(finding terms | clinical history term a, clinical history term b) or higher order. For simplicity purposes, the rest of the description will use the single-term probabilities but a higher order probability can be applied in a similar manner.

When analyzing a current report, recommendations may be generated using the set of associations 1. At the same time, the set of associations 1 may be updated by the association generator 14, for example by updating the conditional probabilities discussed above.

Several different usage schemes may be implemented for the indication of a suggestion of missing concept terms.

In a first usage scheme, post-reporting checking is used. Missing concept terms are indicated after the report has been completed. If missing concept terms are found, the user may decide to update the completed report. For example, for each first concept term (or a combination of first concept terms), the system may use the pre-computed list of significant associations to find a relevant second concept term or terms. A threshold may be determined in advance or chosen by the user (e.g. interaction through a slider mechanism) to limit the list of associations. The completed report may then be analyzed to extract the contained concept terms using NLP techniques. This list of terms from the report is compared to the terms corresponding to the significant associations to only display the missing terms as suggestions. An explanation of why a particular term is suggested may also be indicated to the user, for example by showing the first concept term and the strength of the association.

The displayed list of suggested terms can be sorted based on the conditional probabilities calculated, or based on a strength measure of the association computed in a different way. The post-reporting checking can be done right after completion of the report or off-line. In the latter case, the flagged reports having missing terms can be brought later to the attention of the radiologist or selected for peer review.

Other types of correlations can be identified, such as those between two findings concepts or between a finding concept and a diagnostic concept. These concepts were produced by the radiologist and bring key contextual information that could be used to attach more weight to the medical concept terms and narrow down the list of suggested terms. As an example, if a suggested term is a finding "a" and if another finding 'b' is already in the report, a high probability P (finding term "a" | finding term "b") may promote the finding 'a" towards the top of the list of suggested terms.

In a second usage scheme, reporting assistance is provided by suggesting medical terms during the creation of the report. This scenario differs from the previous one as the suggested terms are offered on the fly when the radiologist is typing or dictating the report. This gives the doctor the possibility to go back to images for further scrutiny. This functionality can be used, for example, for training purposes, either for resident physicians or for continuous medical education.

In a third usage scheme, a region recommender based on image viewer 15 helps the user to quickly find relevant image portions corresponding to the missing second concept terms. Based on the first concept terms, for example extracted from a clinical history leading the imaging exam, the user may be presented with a list of regions using the method described above. The interaction with the image may comprise the use of image processing techniques to segment the image (mesh model) and an atlas to map each region with the corresponding region concepts. As a result, upon clicking on one of the suggested regions in the list (the list may be sorted by relevance using the conditional probabilities), the corresponding region may be highlighted.

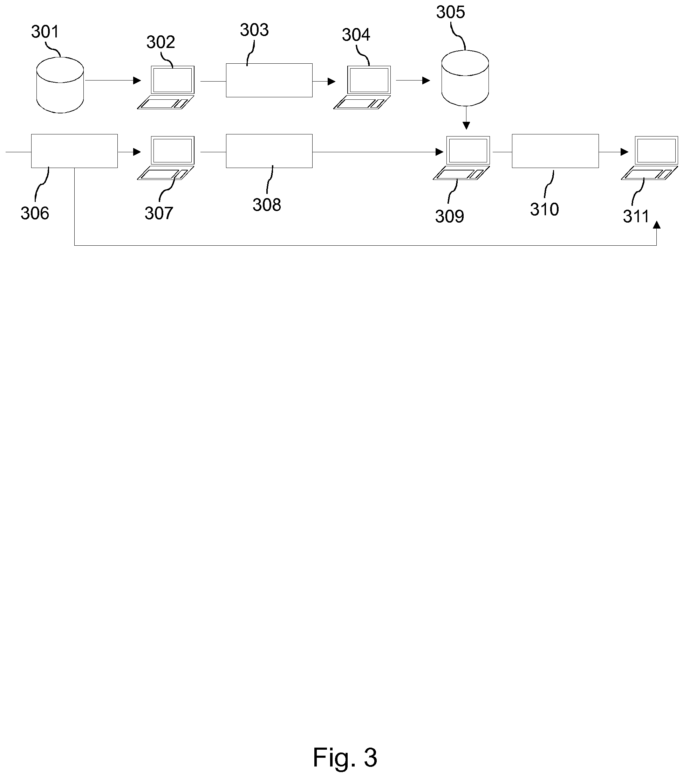

Fig. 3 illustrates another example of a system for aiding report authoring. The components of this system are to a large extent similar to the system depicted in Fig. 1. Prior reports from a database 301 may be analyzed using an extraction subsystem 302 which converts the raw text into extracted report terms 303. This extraction subsystem 302 may be similar to the concept term extractor 2, and arranged for being applied to the prior reports from database 301. The correlation discovery subsystem 304 looks over the extracted report terms 303 from a large number of reports in the database and identifies the significant associations. These can be stored conceptually in a database of significant correlations 305, similar to the set of associations 1. The correlation discovery subsystem 304 may be similar to the association generator 14. When a new report 306 (similar to the at least partially completed report 5) is being considered, the same or a different extraction subsystem 307 (which may be similar to the concept term extractor 2) is executed to convert the raw report 306 (e.g. the patient history) into extracted report terms 308. These extracted report terms 308 may be similar to the at least one first concept term 6. These extracted report terms 308 are entered into a recommendation subsystem 309 (which may be similar to the missing concept finder 3) which uses the discovered term associations or correlations 305 to produce a list of new recommended terms 310, which may be similar to the at least one second concept term 11. These new recommended terms 310 may be displayed via display subsystem 311 (which may be similar to the indicator 4) by applying one or more of the functionalities described above. Note that the various subsystems and databases mentioned in this description may be part of a single physical computer system or of multiple physical computer systems.

It will be appreciated that the invention also applies to computer programs, particularly computer programs on or in a carrier, adapted to put the invention into practice. The program may be in the form of a source code, an object code, a code intermediate source and object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing step of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a storage medium, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A computer-implemented system for extracting and displaying a missing concept term to the author of a medical diagnosis or treatment report (5) when the author is editing the report (5), comprising

   - a set of stored associations (1), wherein each association links at least one first concept term (6) in similar reports in a medical knowledge domain with at least one second concept term (11) in the similar reports, wherein the association indicates a frequent co-occurrence of the at least one first concept term and the at least one second concept term in those reports;
   - a concept term extractor (2) arranged for extracting the at least one first concept term (6) from an at least partially completed report (5) relating to a particular patient being edited in the system by an author, the report (5) being comprised in a data record (7) relating to the particular patient and stored in the system;
   - a missing concept term finder (3) arranged for checking whether at least one second concept term (11) linked to the at least one first concept term (6) based on the set of associations (1) is missing from the at least partially completed report (5), and if so outputting the at least one missing second concept term (11);
   - an indicator (4) arranged for displaying to the author the at least one second concept term (11) that is output by the missing concept term finder (3), when the author is editing the report (5);

- and a report creation tool (10) for enabling the author to create at least part of the report (5), wherein the indicator (4) is arranged for displaying the at least one second concept term (11) during a process of creating the at least part of the report (5);

- wherein the at least one second concept term (11) comprises an anatomical region term, and wherein the system further comprises an image viewer (15) configured to indicate to the author a view of an anatomical region of the patient based on the anatomical region term, wherein the anatomical region corresponds to the anatomical region term.

2. The system according to claim 1, wherein the concept term extractor (2) is arranged for extracting at least one first concept term (6) from medical history information (8) relating to the patient comprised in the data record (7).

3. The system according to claim 1 or 2, wherein the set of associations (1) comprises associations linking first concept terms (6) belonging to a first section of the similar reports to second concept terms (11) belonging to a different second section of the reports, and the concept term extractor (2) is arranged to extract the at least one first concept term (6) from a first section of the at least partially completed report (5).

4. The system according to claim 3, wherein the at least one second concept term (11) is associated with a particular section of the report, and the indicator (4) is arranged for indicating with which section the at least one second concept term (11) is associated.

5. The system according to claim 3 or 4, wherein the indicator (4) is arranged for displaying to the author the at least one second concept term (11) when the author is editing the section associated therewith.

6. The system according to any preceding claim, wherein the system comprises a user interface element (9) for enabling the author to indicate that the report (5) is complete, and wherein the indicator (4) is arranged for indicating the at least one second concept term (11) that is output by the missing concept term finder (3), in response to the author indication that the report (5) is complete.

7. The system according to any preceding claim, further comprising a second concept term classifier (12) arranged for classifying the at least one second concept term (11) by type of concept, and wherein the indicator (4) is arranged for indicating the type of concept for a second concept term (11).

8. The system according to any preceding claim, wherein the associations of the set of associations (1) have different strengths, and further comprising a second concept term sorter (13) arranged for sorting the at least one second concept term (11) found by the missing concept term finder (3), based on the strengths of the associations by which the at least one second concept term (11) was found.

9. The system according to any preceding claim, further comprising an association generator (14) arranged for creating an association based on co-occurrence frequencies of first and second concept terms in a collection of reports.

10. A workstation comprising the system according to any preceding claim.

11. A computer-implemented method of extracting and displaying a missing concept term to the author of a medical diagnosis or treatment report (5) when the author is editing the report (5), comprising

- storing associations (1), wherein each association links at least one first concept term (6) in similar reports in a medical knowledge domain with at least one second concept term (11) in the similar reports, wherein the association indicates a frequent co-occurrence of the at least one first concept term and the at least one second concept term in those reports;

- extracting the at least one first concept term (6) from an at least partially completed report (5) relating to a particular patient being edited in the system by an author, the report (5) being comprised in a data record (7) relating to the particular patient and stored in the system;

- checking whether at least one second concept term (11) linked to the at least one first concept term (6) based on the set of associations (1) is missing from the at least partially completed report (5), and if so outputting the at least one missing second concept term (11);

- displaying to the author the at least one second concept term (11) that is output by the missing concept term finder (3), when the author is editing the report (5);

- and providing a report creation tool (10) for enabling the author to create at least part of the report (5), wherein the method comprises displaying the at least one second concept term (11) during a process of creating the at least part of the report (5);

wherein the at least one second concept term (11) comprises an anatomical region term, and wherein the system further comprises an image viewer (15) configured to indicate to the author a view of an anatomical region of the patient based on the anatomical region term, wherein the anatomical region corresponds to the anatomical region term.

**12.** A computer program product comprising instructions for causing a processor system to perform the method according to claim 11.

**Patentansprüche**

**1.** Computerumgesetztes System zum Entnehmen einer fehlenden Begrifflichkeit und zum Anzeigen derselben für den Verfasser eines medizinischen Diagnose- oder Behandlungsberichts (5), wenn der Verfasser den Bericht (5) überarbeitet, umfassend

- einen Satz von gespeicherten Verknüpfungen (1), wobei jede Verknüpfung mindestens eine erste Begrifflichkeit (6) in ähnlichen Berichten auf einem medizinischen Wissensgebiet mit mindestens einer zweiten Begrifflichkeit (11) in den ähnlichen Berichten verlinkt, wobei die Verknüpfung ein häufiges gleichzeitiges Vorkommen der mindestens einen ersten Begrifflichkeit und der mindestens einen zweiten Begrifflichkeit in diesen Berichten bedeutet;
- eine Vorrichtung (2) zum Entnehmen von Begrifflichkeiten, die eingerichtet ist, um die mindestens eine erste Begrifflichkeit (6) aus einem mindestens teilweise fertiggestellten Bericht (5) bezüglich eines bestimmten Patienten, der in dem System von einem Verfasser überarbeitet wird, zu entnehmen, wobei der Bericht (5) in einem Datensatz (7) bezüglich des bestimmten Patienten enthalten und in dem System gespeichert ist;
- eine Vorrichtung (3) zum Finden von fehlenden Begrifflichkeiten, die eingerichtet ist, um zu überprüfen, ob mindestens eine zweite Begrifflichkeit (11), die mit der mindestens einen ersten Begrifflichkeit (6) basierend auf dem Satz von Verknüpfungen (1) verlinkt ist, in dem mindestens teilweise fertiggestellten Bericht (5) fehlt, und wenn ja, zum Ausgeben der mindestens einen fehlenden zweiten Begrifflichkeit (11);
- einen Indikator (4), der eingerichtet ist, um für den Verfasser die mindestens eine zweite Begrifflichkeit (11), die durch die Vorrichtung (3) zum Finden von fehlenden Begrifflichkeiten ausgegeben wird, anzuzeigen, wenn der Verfasser den Bericht (5) überarbeitet;
- und ein Berichterstellungs-Tool (10), damit der Verfasser mindestens einen Teil des Berichts (5) erstellen kann, wobei der Indikator (4) eingerichtet ist, um die mindestens eine zweite Begrifflichkeit (11) während eines Prozesses des Erstellens des mindestens einen Teils des Berichts (5) anzuzeigen;
- wobei die mindestens eine zweite Begrifflichkeit (11) eine Benennung einer anatomischen Region umfasst, und wobei das System ferner eine Bildbetrachtungsvorrichtung (15) umfasst, die konfiguriert ist, um dem Verfasser eine Ansicht einer anatomischen Region des Patienten basierend auf der Benennung der anatomischen Region anzugeben, wobei die anatomische Region der Benennung der anatomischen Region entspricht.

**2.** System nach Anspruch 1, wobei die Vorrichtung (2) zum Entnehmen von Begrifflichkeiten eingerichtet ist, um mindestens eine erste Begrifflichkeit (6) aus der Anamnese (8) bezüglich des Patienten, die in dem Datensatz (7) enthalten ist, zu entnehmen.

**3.** System nach Anspruch 1 oder 2, wobei der Satz von Verknüpfungen (1) Verknüpfungen umfasst, die erste Begrifflichkeiten (6), die zu einem ersten Abschnitt der ähnlichen Berichte gehören, mit zweiten Begrifflichkeiten (11), die zu einem anderen zweiten Abschnitt der Berichte gehören, verlinken, und die Vorrichtung (2) zum Entnehmen von Begrifflichkeiten eingerichtet ist, um die mindestens eine erste Begrifflichkeit (6) aus einem ersten Abschnitt des mindestens teilweise fertiggestellten Berichts (5) zu entnehmen.

**4.** System nach Anspruch 3, wobei die mindestens eine zweite Begrifflichkeit (11) mit einem bestimmten Abschnitt des Berichts verknüpft ist, und der Indikator (4) eingerichtet ist, um anzugeben, mit welchem Abschnitt die mindestens eine zweite Begrifflichkeit (11) verknüpft ist.

**5.** System nach Anspruch 3 oder 4, wobei der Indikator (4) eingerichtet ist, um für den Verfasser die mindestens eine zweite Begrifflichkeit (11) anzuzeigen, wenn der Verfasser den damit verknüpften Abschnitt überarbeitet.

**6.** System nach einem der vorhergehenden Ansprüche, wobei das System ein Benutzerschnittstellenelement (9) umfasst, damit der Verfasser angeben kann, dass der Bericht (5) fertiggestellt ist, und wobei der Indikator (4) eingerichtet ist, um die mindestens eine zweite Begrifflichkeit (11), die durch die Vorrichtung (3) zum Finden von fehlenden Begrifflichkeiten ausgegeben wird, als Reaktion auf die Angabe des Verfassers, dass der Bericht (5) fertiggestellt ist, anzugeben.

**7.** System nach einem der vorhergehenden Ansprüche, ferner umfassend eine Vorrichtung (12) zum Klassifizieren von zweiten Begrifflichkeiten, die eingerichtet ist, um die mindestens eine zweite Begrifflichkeit (11) nach Begriffstyp zu klassifizieren, und wobei der Indikator (4) eingerichtet ist, um den Begriffstyp für eine zweite Begrifflichkeit (11) anzugeben.

**8.** System nach einem der vorhergehenden Ansprüche, wobei die Verknüpfungen des Satzes von Verknüpfungen (1) unterschiedliche Stärken aufweisen, und ferner umfassend eine Vorrichtung (13) zum Sortieren von zweiten Begrifflichkeiten, die eingerichtet ist, um die mindestens eine zweite Begrifflichkeit (11), die durch die Vorrichtung (3) zum Finden von fehlenden Begrifflichkeiten gefunden wurde, basierend auf den Stärken der Verknüpfungen, durch welche die mindestens eine zweite Begrifflichkeit (11) gefunden wurde, zu sortieren.

**9.** System nach einem der vorhergehenden Ansprüche, ferner umfassend eine Vorrichtung (14) zum Generieren von Verknüpfungen, die eingerichtet ist, um basierend auf der Häufigkeit des gleichzeitigen Vorkommens von ersten und zweiten Begrifflichkeiten in einer Sammlung von Berichten eine Verknüpfung zu erstellen.

**10.** Arbeitsstation, umfassend das System nach einem der vorhergehenden Ansprüche.

**11.** Computerumgesetztes Verfahren zum Entnehmen einer fehlenden Begrifflichkeit und zum Anzeigen derselben für den Verfasser eines medizinischen Diagnose- oder Behandlungsberichts (5), wenn der Verfasser den Bericht (5) überarbeitet, umfassend folgende Schritte

- Speichern von Verknüpfungen (1), wobei jede Verknüpfung mindestens eine erste Begrifflichkeit (6) in ähnlichen Berichten in einem medizinischen Wissensgebiet mit mindestens einer zweiten Begrifflichkeit (11) in den ähnlichen Berichten verlinkt, wobei die Verknüpfung ein häufiges gleichzeitiges Vorkommen der mindestens einen ersten Begrifflichkeit und der mindestens einen zweiten Begrifflichkeit in diesen Berichten bedeutet;
- Entnehmen der mindestens einen ersten Begrifflichkeit (6) aus einem mindestens teilweise fertiggestellten Bericht (5) bezüglich eines bestimmten Patienten, der in dem System von einem Verfasser überarbeitet wird, wobei der Bericht (5) in einem Datensatz (7) bezüglich des bestimmten Patienten enthalten und in dem System gespeichert ist;
- Überprüfen, ob mindestens eine zweite Begrifflichkeit (11), die mit der mindestens einen ersten Begrifflichkeit (6) basierend auf dem Satz von Verknüpfungen (1) verlinkt ist, in dem mindestens teilweise fertiggestellten Bericht (5) fehlt, und wenn ja, Ausgeben der mindestens einen fehlenden zweiten Begrifflichkeit (11);
- Anzeigen für den Verfasser der mindestens einen zweiten Begrifflichkeit (11), die durch die Vorrichtung (3) zum Finden von fehlenden Begrifflichkeiten ausgegeben wird, wenn der Verfasser den Bericht (5) überarbeitet;
- und Bereitstellen eines Berichterstellungs-Tools (10), damit der Verfasser mindestens einen Teil des Berichts (5) erstellen kann, wobei das Verfahren das Anzeigen der mindestens einen zweiten Begrifflichkeit (11) während eines Prozesses des Erstellens des mindestens einen Teils des Berichts (5) umfasst;
wobei die mindestens eine zweite Begrifflichkeit (11) eine Benennung einer anatomischen Region umfasst, und wobei das System ferner eine Bildbetrachtungsvorrichtung (15) umfasst, die konfiguriert ist, um für den Verfasser eine Ansicht einer anatomischen Region des Patienten basierend auf der Benennung der anatomischen Region anzugeben, wobei die anatomische Region der Benennung der anatomischen Region entspricht.

**12.** Computerprogrammprodukt, umfassend Anweisungen, um zu bewirken, dass ein Prozessorsystem das Verfahren nach Anspruch 11 ausführt.

**Revendications**

**1.** Système mis en oeuvre par ordinateur pour l'extraction et l'affichage d'un terme de concept manquant à l'auteur d'un rapport de diagnostic ou de traitement médical (5) lorsque l'auteur modifie le rapport (5), comprenant

- un ensemble d'associations mémorisées (1), dans lequel chaque association relie au moins un premier terme de concept (6) dans des rapports similaires dans un domaine de connaissances médicales à au moins un deuxième terme de concept (11) dans les rapports similaires, dans lequel l'association indique une cooccurrence fréquente de l'au moins un premier terme de concept et de l'au moins un deuxième terme de concept dans ces rapports ;

- un extracteur de terme de concept (2) agencé pour l'extraction de l'au moins un premier terme de concept (6) d'un rapport au moins partiellement rempli (5) relatif à un patient particulier en cours de modification dans le système par un auteur, le rapport (5) étant compris dans un enregistrement de données (7) relatif au patient particulier et mémorisé dans le système ;

- un détecteur de terme de concept manquant (3) agencé pour le contrôle si au moins un deuxième terme de concept (11) lié à l'au moins un premier terme de concept (6) sur la base de l'ensemble d'associations (1) est ou non manquant dans le rapport au moins partiellement rempli (5), et, si tel est le cas, la délivrance de l'au moins un deuxième terme de concept manquant (11) ;

- un indicateur (4) agencé pour l'affichage, à l'auteur, de l'au moins un deuxième terme de concept (11) qui est délivré par le détecteur de terme de concept manquant (3), lorsque l'auteur modifie le rapport (5) ;

- et un outil de création de rapport (10) pour permettre à l'auteur de créer au moins une partie du rapport (5), dans lequel l'indicateur (4) est agencé pour l'affichage de l'au moins un deuxième terme de concept (11) au cours d'un processus de création de l'au moins une partie du rapport (5) ;

- dans lequel l'au moins un deuxième terme de concept (11) comprend un terme de région anatomique, et dans lequel le système comprend en outre une visionneuse d'images (15) configurée pour l'indication, à l'auteur, d'une vue d'une région anatomique du patient sur la base du terme de région anatomique, dans lequel la région anatomique correspond au terme de région anatomique.

2. Système selon la revendication 1, dans lequel l'extracteur de terme de concept (2) est agencé pour l'extraction d'au moins un premier terme de concept (6) à partir d'informations d'historique médical (8) relatives au patient comprises dans l'enregistrement de données (7).

3. Système selon la revendication 1 ou 2, dans lequel l'ensemble d'associations (1) comprend des associations reliant des premiers termes de concept (6) appartenant à une première section des rapports similaires à des deuxièmes termes de concept (11) appartenant à une deuxième section différente des rapports, et l'extracteur de terme de concept (2) est agencé pour l'extraction de l'au moins un premier terme de concept (6) d'une première section du rapport au moins partiellement rempli (5).

4. Procédé selon la revendication 3, dans lequel l'au moins un deuxième terme de concept (11) est associé à une section particulière du rapport, et l'indicateur (4) est agencé pour indiquer à quelle section l'au moins un deuxième terme de concept (11) est associé.

5. Procédé selon la revendication 3 ou 4, dans lequel l'indicateur (4) est agencé pour l'affichage, à l'auteur, de l'au moins un deuxième terme de concept (11) lorsque l'auteur modifie la section qui lui est associée.

6. Système selon l'une quelconque des revendications précédentes, dans lequel le système comprend un élément d'interface utilisateur (9) pour permettre à l'auteur d'indiquer que le rapport (5) est rempli, et dans lequel l'indicateur (4) est agencé pour l'indication de l'au moins un deuxième terme de concept (11) qui est délivré par le détecteur de terme de concept manquant (3), en réponse à l'indication, à l'auteur, que le rapport (5) est rempli.

7. Système selon l'une quelconque des revendications précédentes, comprenant en outre un classificateur de deuxième terme de concept (12) agencé pour la classification de l'au moins un deuxième terme de concept (11) par type de concept, et dans lequel l'indicateur (4) est agencé pour l'indication du type de concept pour un deuxième terme de concept (11).

8. Système selon l'une quelconque des revendications précédentes, dans lequel les associations de l'ensemble d'associations (1) ont des forces différentes, et comprenant en outre une trieuse de deuxième terme de concept (13) agencée pour le tri de l'au moins un deuxième terme de concept (11) trouvé par le détecteur de terme de concept manquant (3), sur la base des forces des associations par lesquelles l'au moins un deuxième terme de concept (11) a été trouvé.

9. Système selon l'une quelconque des revendications précédentes, comprenant en outre un générateur d'association (14) agencé pour la création d'une association sur la base de fréquences de cooccurrence de premier et deuxième termes de concept dans un recueil de rapports.

**10.** Poste de travail comprenant le système selon l'une quelconque des revendications précédentes.

**11.** Procédé mis en oeuvre par ordinateur permettant l'extraction et l'affichage d'un terme de concept manquant à l'auteur d'un rapport de diagnostic ou de traitement médical (5) lorsque l'auteur modifie le rapport (5), comprenant

- la mémorisation d'associations (1), dans lequel chaque association relie au moins un premier terme de concept (6) dans des rapports similaires dans un domaine de connaissances médicales à au moins un deuxième terme de concept (11) dans les rapports similaires, dans lequel l'association indique une cooccurrence fréquente de l'au moins un premier terme de concept et de l'au moins un deuxième terme de concept dans ces rapports ;
- l'extraction de l'au moins un premier terme de concept (6) d'un rapport au moins partiellement rempli (5) relatif à un patient particulier en cours de modification dans le système par un auteur, le rapport (5) étant compris dans un enregistrement de données (7) relatif au patient particulier et mémorisé dans le système ;
- le contrôle si au moins un deuxième terme de concept (11) lié à l'au moins un premier terme de concept (6) sur la base de l'ensemble d'associations (1) est ou non manquant dans le rapport au moins partiellement rempli (5), et, si tel est le cas, la délivrance de l'au moins un deuxième terme de concept manquant (11) ;
- l'affichage, à l'auteur, de l'au moins un deuxième terme de concept (11) qui est délivré par le détecteur de terme de concept manquant (3), lorsque l'auteur modifie le rapport (5) ;
- et la fourniture d'un outil de création de rapport (10) pour permettre à l'auteur de créer au moins une partie du rapport (5), dans lequel le procédé comprend l'affichage de l'au moins un deuxième terme de concept (11) au cours d'un processus de création de l'au moins une partie du rapport (5) ;
dans lequel l'au moins un deuxième terme de concept (11) comprend un terme de région anatomique, et dans lequel le système comprend en outre une visionneuse d'images (15) configurée pour l'indication, à l'auteur, d'une vue d'une région anatomique du patient sur la base du terme de région anatomique, dans lequel la région anatomique correspond au terme de région anatomique.

**12.** Produit de programme informatique comprenant des instructions pour amener un système de processeur à effectuer le procédé selon la revendication 11.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050065813 A

**Non-patent literature cited in the description**

- **GEIS, J.** Medical Imaging Informatics: How It Improves Radiology Practice Today. *Journal of Digital Imaging,* 2007, vol. 20 (2), 99